(19) Europäisches Patentamt / European Patent Office / Office européen des brevets

(11) **EP 4 722 764 A1**

(12) **DEMANDE DE BREVET EUROPEEN**

(43) Date de publication:
08.04.2026 Bulletin 2026/15

(21) Numéro de dépôt: 25203776.7

(22) Date de dépôt: 22.09.2025

(51) Classification Internationale des Brevets (IPC):
$G01V\ 3/12^{(2006.01)}$    $G01S\ 13/88^{(2006.01)}$
$G01V\ 3/15^{(2006.01)}$    $G01V\ 3/38^{(2006.01)}$

(52) Classification Coopérative des Brevets (CPC):
G01V 3/12; G01S 13/885; G01V 3/15; G01V 3/38

(84) Etats contractants désignés:
AL AT BE BG CH CY CZ DE DK EE ES FI FR GB GR HR HU IE IS IT LI LT LU LV MC ME MK MT NL NO PL PT RO RS SE SI SK SM TR
Etats d'extension désignés:
BA
Etats de validation désignés:
GE KH LA MA MD TN

(30) Priorité: 02.10.2024 FR 2410589

(71) Demandeur: Commissariat à l'Energie Atomique et aux Energies Alternatives
75015 Paris (FR)

(72) Inventeurs:
• PAGANI, Pascal
  38054 GRENOBLE (FR)
• DORE, Jean-Baptiste
  38054 GRENOBLE (FR)
• D'ERRICO, Raffaele
  38054 GRENOBLE (FR)

(74) Mandataire: Atout PI Laplace
Immeuble Up On
25 Boulevard Romain Rolland
CS 40072
75685 Paris Cedex 14 (FR)

(54) **METHODE DE DETERMINATION DE PROPRIETES RADIOELECTRIQUES D'UN MILIEU STRATIFIE AU MOYEN D'UN SYSTEME DE DETECTION RADIOFREQUENCES**

(57) Méthode de détermination de propriétés radioélectriques d'un milieu stratifié comprenant les étapes de :
- Déterminer (101), au moins une mesure d'une fonction de transfert fréquentielle d'un canal de transmission caractérisant le milieu,
- Déterminer (102) un modèle de ladite fonction de transfert fréquentielle pour un modèle du milieu composé d'au moins deux couches séparées par un plan,
- Déterminer (103,104) une fonction d'estimation d'un coefficient de corrélation entre l'au moins une mesure d'une fonction de transfert fréquentielle et le modèle de ladite fonction,
- Rechercher (105) au moins un maximum local de la fonction d'estimation
- En déduire (106) au moins une valeur de position d'un plan et une valeur associée à ce plan d'une variable caractérisant la propriété radioélectrique du milieu.

[Fig. 1]

FIG.1

## Description

L'invention concerne le domaine des dispositifs et méthodes non destructives permettant de caractériser la composition d'un matériau ou d'un milieu non homogène au moyen d'un système radiofréquence. Par exemple, l'invention concerne notamment les radars à pénétration de sol qui permettent de détecter, localiser ou identifier des cibles souterraines. L'invention s'applique également à la caractérisation d'autres milieux tels que les épaisseurs de peau, graisse et muscle d'un être vivant (humain ou animal) au moyen d'un système de détection radiofréquence positionné à la surface de la peau.

L'invention porte plus précisément sur une méthode de détermination de propriétés radioélectriques d'un milieu non homogène stratifié tel que le sol ou les tissus biologiques au moyen d'un système de détection radiofréquence

Les méthodes de détection de cibles enterrées à l'aide d'un radar à pénétration de sol sont généralement basées sur des algorithmes qui nécessitent, dans leurs paramètres, une estimation précise des permittivités diélectriques des différentes couches du sol. En général, ces informations ne sont pas connues ou sont estimées grossièrement sous la forme d'une valeur moyenne pour l'ensemble de la structure du sol. Ces approximations peuvent augmenter les incertitudes sur les résultats de détection et caractérisation des cibles souterraines.

Il y a donc un besoin pour une méthode permettant d'estimer précisément les permittivités relatives des différentes couches du sol ainsi que leurs épaisseurs afin d'utiliser ultérieurement ces informations pour améliorer les algorithmes de détection de cibles au moyen de radars à pénétration de sol.

Dans la suite du texte, l'invention est décrite dans le contexte de la détection de cibles enterrées dans le sol mais l'invention s'applique plus généralement à des cibles situées dans un matériau quelconque qui peut être différent du sol. L'invention s'applique également à l'imagerie médicale par microonde pour détecter, par exemple, des veines sous la peau ou les épaisseurs des différents tissus biologiques, ou encore au contrôle non destructif par microonde pour estimer l'épaisseur de couches de béton d'une structure industrielle.

Pour toutes les applications qui visent à détecter des éléments dans un milieu ou un matériau à partir d'un système de détection radiofréquence, la connaissance des propriétés radioélectriques du milieu de détection est importante.

De façon générale, il est intéressant de déterminer les propriétés radioélectriques d'un objet de propriétés inconnues.

En effet, la localisation spatiale des cibles recherchées est généralement réalisée par l'analyse du temps de propagation des signaux radioélectriques émis et reçus par le système radiofréquence, qui sont ensuite convertis en distances à l'aide d'une estimation de la vitesse de propagation des ondes radioélectriques dans le milieu. Or, la vitesse de propagation d'une onde électromagnétique dépend des propriétés radioélectriques du milieu, et en particulier (bien que pas exclusivement) de sa permittivité diélectrique. Les propriétés radioélectriques du milieu permettent de décrire la réponse de ce milieu à un champ électrique appliqué.

Par exemple, dans le cas de radars à pénétration de sol, le milieu traversé par les ondes électromagnétiques est en général le sol. Pour le cas d'applications dans le domaine de la santé, le milieu traversé concerne un ensemble de tissus biologiques.

La plupart du temps, des hypothèses a priori sont faites sur les valeurs effectives des caractéristiques radioélectriques du milieu telles que la permittivité diélectrique. Or la précision de ces valeurs peut être importante pour obtenir une précision suffisante pour la détection et la caractérisation de cibles.

L'invention répond donc à un problème général de caractérisation précise des propriétés radioélectriques d'un milieu dans lequel une onde électromagnétique se propage, notamment la permittivité diélectrique ou la perméabilité.

Dans le domaine des radars à pénétration de sol, il existe plusieurs méthodes de détection de la position d'une cible.

Une méthode de l'état de l'art connue est l'ajustement de courbe hyperbole décrit par exemple dans la référence [1]. Lorsqu'une antenne du radar à pénétration de sol est déplacée au-dessus de la surface du sol et que l'onde pénétrante rencontre des cibles enterrées ou des interfaces entre différentes couches du sol, le signal reçu observé dans le domaine temporel présente une forme d'hyperbole, en raison des temps de parcours des ondes réfléchies, qui diffèrent suivant la position relative des antennes du radar et de la cible. Plusieurs configurations de positions d'antennes peuvent être utilisées afin d'observer ce phénomène. Par exemple, les antennes d'émission (Tx) et de réception (Rx) peuvent se déplacer d'un mouvement identique, l'une restant ainsi en position fixe par rapport à l'autre. Alternativement, l'antenne Tx peut réaliser un déplacement symétrique à celui de l'antenne Rx par rapport à un point central fixe, cette configuration étant alors nommée « Common Mid Point ». Enfin, une antenne peut se placer à différentes positions par rapport à la seconde antenne fixe, dans une configuration appelée « Wide Angle Reflection and Refraction ».

Toutes ces techniques d'observation permettent d'obtenir un diagramme en forme d'hyperbole lorsqu'on observe le signal reçu dans le domaine temporel en fonction d'une métrique de position des antennes. Le sommet de l'hyperbole indique alors la position effective de la cible (ou de l'interface) et la forme de l'hyperbole dépend du pas horizontal et de la vitesse du signal dans le matériau : plus la vitesse est élevée, plus l'hyperbole est large et inversement.

En analysant la forme de l'hyperbole, on peut remonter à la vitesse de propagation de l'onde dans le milieu et ainsi remonter à la permittivité électrique du milieu. L'ajustement du signal observé sur une hyperbole théorique est réalisé par une analyse de semblance, telle que décrite par exemple dans les références [2, 3].

Cette méthode est très courante pour l'étalonnage de données issues d'un radar à pénétration de sol ou « Ground Penetrating Radar » (GPR) en anglais, mais présente plusieurs inconvénients majeurs. La précision de la méthode reste faible en raison de la difficulté d'ajuster une courbe théorique sur une observation expérimentale entachée d'incertitude. Augmenter la précision nécessite d'acquérir un plus grand nombre d'observations, ce qui implique un déplacement des antennes à chaque observation. Le déplacement des antennes peut être évité si on dispose de plusieurs antennes Tx et Rx réparties spatialement, mais dans ce cas la précision de la technique d'ajustement est faible. La méthode est également peu adaptée à la détection de couches stratifiées pour un milieu non homogène, car les différentes réflexions produisent des hyperboles qui se superposent, rendant l'ajustement plus difficile. Cette situation nécessite de mettre en œuvre des algorithmes complexes, comme l'illustre la référence [4].

Un autre type de méthode connue concerne les méthodes de migration, telles que présentées par exemple dans la référence [5]. Ces méthodes cherchent à réduire l'imprécision de la méthode d'ajustement d'hyperboles due aux incertitudes d'observation du signal reçu. Il s'agit d'une forme de traitement mathématique dont le but principal est d'augmenter la précision des traces hyperboliques des cibles et interfaces. Théoriquement, ce processus permet de réduire les hyperboles acquises par le radar en un point unique de localisation. Cependant, la mise en œuvre de cette technique s'avère délicate lorsqu'on l'applique à des données expérimentales potentiellement bruitées. Il existe une grande variété de méthodes de migration différentes parmi lesquelles la sommation hyperbolique, la migration de Kirchhoff, la focalisation en projection inverse, la migration en décalage de phase, et la migration $\omega$-$k$ [5].

Le principal paramètre requis pour réduire l'hyperbole à un point correspond aux propriétés diélectriques du sol tel qu'enseigné dans la référence [6]. Cela fait du processus de migration un moyen d'augmenter la précision de l'ajustement de la courbe puisque le processus ne fonctionne correctement que si la vitesse de transmission au sol appliquée est précise. Ainsi les méthodes de migration utilisent une estimation moyenne de la permittivité relative d'un matériau pour une profondeur donnée. Un inconvénient de ces méthodes est leur relative imprécision lorsque le matériau est composé de plusieurs couches de permittivités différentes.

La référence [6] propose une méthode appliquée à la détection de cibles enterrées. Elle propose de tester différentes valeurs de permittivités moyennes pour une acquisition donnée. La permittivité sélectionnée est celle qui minimise la surface de la cible détectée. De ce fait, cette méthode n'est pas générique et n'est pas applicable en l'absence de cible, par exemple dans le cas d'un matériau inhomogène stratifié. D'autre part, la métrique de la surface apparente de la cible est peu précise et n'est adaptée qu'aux cibles de forme simple, dont la surface apparente varie de manière monotone avec l'erreur absolue dans la permittivité supposée.

D'autres méthodes connues visent à résoudre la problématique de détermination des propriétés radioélectriques d'un milieu à l'aide d'un système de détection radiofréquence. On peut citer notamment les demandes de brevets et brevets WO2020180191, EP3164672, WO2022091456, FR3041108 et FR3142553.

Toutes ces méthodes présentent des inconvénients, certaines méthodes sont invasives et destructives, d'autres nécessitent la connaissance préalable de la géométrie du milieu, d'autres ne sont pas applicables à un milieu inhomogène. De manière générale, ces méthodes proposent des solutions de traitement de données radar dans le domaine temporel.

Il existe un besoin pour une méthode permettant de caractériser précisément les propriétés radioélectriques d'un milieu au moyen d'un système de détection radiofréquences qui résout les limitations des méthodes de l'art antérieur. La méthode proposée doit notamment être faiblement complexe pour être compatible d'une mise en œuvre dans un système embarqué à ressources limitées.

Il est proposé une nouvelle méthode de caractérisation qui opère dans le domaine fréquentiel et qui présente une complexité diminuée par rapport aux méthodes de l'art antérieur.

La méthode selon l'invention est particulièrement adaptée à la caractérisation de milieux stratifiés composés de plusieurs couches séparées par des plans, chaque couche ayant des propriétés radioélectriques différentes ou plus généralement de milieux pouvant être modélisés, même de façon approximative, par une telle structure stratifiée.

L'invention a pour objet une méthode de détermination de propriétés radioélectriques d'un milieu stratifié comprenant les étapes de :

- Déterminer, au moyen d'un système de détection radiofréquence comprenant au moins un émetteur et un récepteur, pour chaque couple associant un récepteur et un émetteur, au moins une mesure d'une fonction de transfert fréquentielle d'un canal de transmission caractérisant le milieu,

- Déterminer un modèle de ladite fonction de transfert fréquentielle pour un modèle du milieu composé d'au moins deux couches séparées par un plan, le modèle de la fonction de transfert étant fonction d'au moins une variable caractérisant une propriété radioélectrique du milieu et d'au moins une variable caractérisant la position du plan par rapport au système de détection,

- Déterminer une fonction d'estimation d'un coefficient de corrélation entre l'au moins une mesure d'une fonction de transfert fréquentielle et le modèle de ladite fonction,

- Rechercher au moins un maximum local de la fonction d'estimation sur le domaine défini par l'au moins une variable caractérisant la position du plan et l'au moins une variable caractérisant une propriété radioélectrique du milieu,

- En déduire au moins une valeur de position d'un plan et une valeur associée à ce plan de la variable caractérisant la propriété radioélectrique du milieu.

Selon un aspect particulier de l'invention, le plan est parallèle à la surface du milieu, le système de détection est disposé parallèlement à la surface du milieu et l'au moins une variable caractérisant la position du plan comprend la profondeur du plan.

Selon un aspect particulier de l'invention, le plan n'est pas parallèle à la surface du milieu, le système de détection est disposé parallèlement à la surface du milieu ou incliné par rapport à la surface du milieu et l'au moins une variable caractérisant la position du plan comprend la profondeur du plan par rapport à un point du système de détection et l'angle d'inclinaison du plan par rapport à la surface du milieu ou par rapport au système de détection.

Selon un aspect particulier de l'invention, les étapes de déterminer au moins une mesure de la fonction de transfert fréquentielle et de déterminer un modèle de ladite fonction de transfert sont réalisées pour chaque couple associant une antenne d'émission et une antenne de réception du système de détection, la méthode comprenant en outre la détermination d'un coefficient de corrélation entre ladite mesure et ledit modèle pour chacun desdits couples, la fonction d'estimation étant calculée à partir de tous les coefficients de corrélation.

Selon un aspect particulier de l'invention, l'amplitude et la phase du modèle de la fonction de transfert fréquentielle dépendent de la distance entre une antenne d'émission et un point du plan et de la distance entre le point du plan et une antenne de réception.

Selon un aspect particulier de l'invention, la fonction d'estimation du coefficient de corrélation est déterminée comme une moyenne des coefficients de corrélation pour tous les couples et pour une ou plusieurs valeurs de fréquence discrètes.

Selon un aspect particulier de l'invention, chaque coefficient de corrélation est pondéré par un coefficient de pondération prédéfini.

Selon un aspect particulier de l'invention, chaque coefficient de pondération est inversement proportionnel au nombre de couples qui présentent la même disposition géométrique entre l'antenne d'émission, le point du plan et l'antenne de réception que celle du couple associé au coefficient de corrélation.

Selon un aspect particulier de l'invention, l'au moins une variable caractérisant une propriété radioélectrique du milieu est prise parmi : la partie réelle ou la partie imaginaire de la permittivité diélectrique ou de la perméabilité.

Selon un aspect particulier de l'invention, le coefficient de corrélation est déterminé par une méthode d'égalisation de type ZF, MMSE ou MRC.

Dans une variante de réalisation, la méthode comprend une étape de remplacement, dans la fonction d'estimation, de la variable de profondeur par une variable de profondeur électrique $Z_{el} = z.\sqrt{\varepsilon_r'}$, où $\varepsilon_r'$ est la partie réelle de la permittivité diélectrique.

Dans une variante de réalisation, la méthode comprend en outre l'application d'un algorithme de type Dix pour déterminer les permittivités respectives des couches du milieu à partir des permittivités estimées.

Selon un aspect particulier de l'invention, le milieu stratifié est le sol et le système de détection est un radar à pénétration de sol.

L'invention a aussi pour objet un dispositif de détermination de propriétés radioélectriques de couches d'un milieu stratifié comprenant un système de détection radiofréquences comprenant au moins une antenne d'émission et une antenne de réception et une unité de traitement, le dispositif étant configuré pour mettre en œuvre la méthode selon l'invention.

L'invention a aussi pour objet un programme d'ordinateur comprenant des instructions de code qui conduisent le dispositif selon l'invention à exécuter les étapes de la méthode selon l'invention.

L'invention a aussi pour objet un support lisible par ordinateur, sur lequel est enregistré le programme d'ordinateur selon l'invention.

D'autres caractéristiques et avantages de la présente invention apparaîtront mieux à la lecture de la description qui suit en relation aux dessins annexés suivants.

[Fig. 1] représente un organigramme détaillant les étapes de mise en œuvre d'une méthode de détermination de propriétés radioélectriques d'un milieu selon un mode de réalisation de l'invention,

[Fig. 2] schématise un exemple de système de détection radiofréquence, apte à mettre en œuvre l'invention,

[Fig. 3] illustre un schéma de principe d'un modèle de milieu stratifié,

[Fig. 4a] représente une illustration de l'estimation de la permittivité par focalisation dans le domaine $(\varepsilon'_r,z)$,

[Fig. 4b] représente une illustration de l'estimation de la permittivité par focalisation dans le domaine $(\varepsilon'_r,z_{el})$,

[Fig. 5a] illustre un exemple d'application de l'invention à un sol stratifié,

[Fig. 5b] illustre un résultat de focalisation pour l'exemple de la figure 4a,

[Fig. 6] représente un schéma de principe d'un modèle de milieu stratifié avec un plan incliné par rapport à la surface du sol,

[Fig. 7] représente un schéma illustrant une variante de réalisation de l'invention,

[Fig. 8a] représente un résultat de focalisation obtenu via la méthode selon l'invention sans application de coefficients de pondération dans le calcul de la fonction d'estimation globale,

[Fig. 8b] représente un résultat de focalisation obtenu via la méthode selon l'invention avec application de coefficients de pondération dans le calcul de la fonction d'estimation globale,

[Fig. 9] représente un diagramme comparatif du maximum de la fonction d'estimation globale en fonction de la partie réelle de la permittivité diélectrique pour les deux variantes des figures 8a et 8b,

[Fig. 10] schématise un exemple de système apte à mettre en œuvre l'invention.

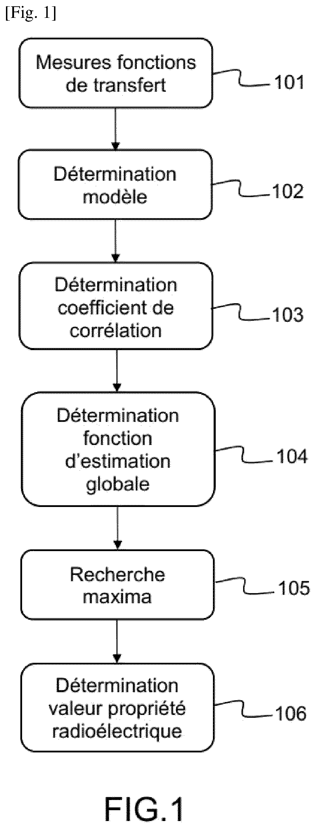

La figure 1 représente un organigramme d'une méthode de détermination de propriétés radioélectriques d'un milieu stratifié selon un mode de réalisation de l'invention.

Dans la suite de la description, la méthode est décrite pour un exemple non limitatif destiné à la détermination de la permittivité d'un milieu tel que le sol au moyen d'un système de détection radiofréquences tel qu'un radar à pénétration de sol. Cependant l'invention n'est pas limitée à la détermination de la permittivité mais s'étend à la détermination de toute propriété radioélectrique caractérisant un milieu tel qu'également la perméabilité.

De même l'invention, n'est pas limitée à l'emploi d'un dispositif radar à pénétration de sol mais s'étend à tout dispositif de détection radiofréquence.

La méthode débute à l'étape 101 avec un ensemble de mesures de fonctions de transfert fréquentielles au moyen d'un dispositif de détection radiofréquence.

Le système de détection radiofréquence est composé d'un émetteur capable d'émettre un signal radiofréquence via au moins une antenne d'émission Tx et d'un récepteur capable de recevoir ce signal radiofréquence via au moins une antenne de réception Rx. Plusieurs versions du signal transmis sont observées et dépendent des positions de l'antenne Tx et de l'antenne Rx.

Selon une variante de réalisation, ces différentes versions du signal reçu sont obtenues à partir de plusieurs antennes Tx situées à des positions fixes et plusieurs antennes Rx situées à des positions fixes, selon une configuration du dispositif de détection radiofréquence appelée « Multiple Input Multiple Output (MIMO) ».

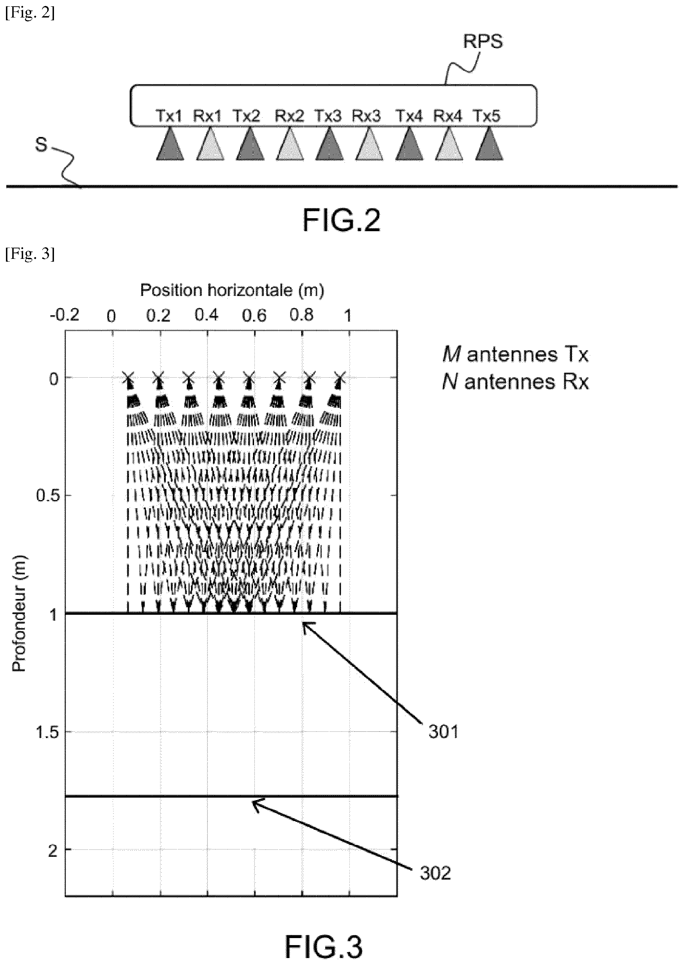

La figure 2 schématise un exemple d'un tel système de détection radiofréquence sous la forme d'un radar à pénétration de sol ayant cinq antennes en émission et quatre antennes en réception.

Alternativement les différentes mesures peuvent également être obtenues en réalisant des transmissions successives, et en déplaçant les antennes Tx ou Rx entre chaque transmission.

Ainsi, on obtient une mesure pour chaque couple associant une antenne d'émission Tx à une antenne de réception Rx pour une position donnée de ces deux antennes.

L'invention s'applique également au cas où une seule antenne sert à la fois en émission et en réception et est connectée à l'émetteur et au récepteur par l'intermédiaire d'un dispositif de séparation tel qu'un coupleur.

La séparation entre les signaux transmis via chaque paire d'antennes Tx et Rx est réalisée dans le domaine temporel à travers des transmissions successives, mais elle peut également être réalisée par toute méthode d'accès multiple classique, comme la séparation en fréquence ou par codes.

On se place à présent dans le cas général d'un système composé de M antennes en émission et N antennes en réception ou plus généralement de M positions d'antennes différentes en émission et N positions d'antennes différentes en réception. M et N sont deux entiers strictement positifs.

Le système de détection radiofréquences comporte en outre un module d'estimation configuré pour déterminer, à partir du signal transmis via l'antenne Tx en position m et reçu via l'antenne Rx en position n, une fonction de transfert complexe $H_{mn}(f)$ pour différentes valeurs de fréquences f.

Plusieurs méthodes d'obtention de la fonction de transfert $H_{mn}(f)$ et de choix d'un signal à transmettre existent dans l'état de l'art et sont des techniques connues du domaine de l'estimation du canal ou du sondage radioélectrique.

Les références [9], [10] et [11] donnent des exemples de telles méthodes.

La fonction de transfert $H_{mn}(f)$ dépend de la composition du milieu traversé par le signal, et en particulier de la répartition spatiale des propriétés radioélectriques du milieu, et dans le cas de la présence de cibles, de la position et de la Surface Equivalente Radar (SER) des cibles.

A l'issue de l'étape 101, on a donc une mesure de fonction de transfert $H_{mn}(f)$ pour plusieurs valeurs de fréquences et pour chaque couple (m,n) associant une antenne en émission et une antenne en réception pour lesquelles une mesure a été réalisée.

A l'étape 102, on détermine ensuite un modèle théorique de la fonction de transfert fréquentielle qui prend en compte les propriétés radioélectriques du milieu traversé par le signal.

Le modèle choisi est adapté à un milieu stratifié composé d'au moins deux couches, chaque couche étant séparée par un plan P situé à une profondeur $z_p$, chaque couche étant constituée d'un milieu homogène. Par exemple, le modèle choisi peut considérer un milieu homogène de permittivité de partie réelle $\varepsilon'_r$ et de partie imaginaire négligeable. La fonction de transfert entre l'émetteur m et le récepteur n, pour ce modèle de milieu stratifié, est modélisée par l'équation (1) suivante :

$$W_{mn}(f) = \frac{\sqrt{G_m G_n}.c}{f.\Delta_P^{mn}(r_m,P).\Delta_P^{mn}(r_n,P).(4\pi)^{3/2}.\sqrt{\varepsilon'_r}} . e^{-i2\pi f \frac{\sqrt{\varepsilon'_r}}{c}(\Delta_P^{mn}(r_m,P)+\Delta_P^{mn}(r_n,P))} \quad (1)$$

$r_m$ et $r_n$ sont respectivement les positions de l'antenne d'émission et de l'antenne de réception,
$G_m$ et $G_n$ sont les gains respectifs des antennes d'émission et de réception,
c est la vitesse de la lumière dans le vide,

$\Delta_P^{mn}(r_m, P)$ et $\Delta_P^{mn}(r_n, P)$ représentent respectivement les distances, selon une géométrique de réflexion spéculaire, entre l'antenne d'émission m et l'antenne de réception n via le plan P, respectivement entre le pont $r_m$ et le plan P et entre le point $r_n$ et le plan P.

Dans le cas où les antennes du radar sont alignées selon l'axe (O x) avec $x_m$ l'abscisse de l'antenne d'émission m et $x_n$ l'abscisse de l'antenne de réception n, les distances se calculent via la relation suivante :

$$\Delta_P^{mn}(r_m,P) = \Delta_P^{mn}(r_n,P) = \sqrt{(\frac{x_n - x_m}{2})^2 + z_P^2} \qquad (2)$$

Ainsi les dimensions spatiales du problème de détection ainsi posé sont réduites à la profondeur selon l'axe z. Le modèle donné par l'équation (1) considère d'une part le déphasage lié à la géométrie de la scène pour une propagation rectiligne et d'autre part une amplitude donnée par l'équation radar issue de la littérature (voir référence [7]).

Sans sortir du cadre de l'invention, d'autres modèles peuvent être développés en remplacement de celui de l'équation (1) dans la mesure où ils prennent en compte au moins une propriété radioélectrique du milieu, par exemple la permittivité $\varepsilon'_r$.

Par exemple, l'équation (1) peut être remplacée par l'équation (1bis) suivante dans laquelle le coefficient $\sqrt{\varepsilon'_r}$ est remplacé par $\varepsilon'_r$.

$$W_{mn}(f) = \frac{\sqrt{G_m G_n}.c}{f.\Delta_P^{mn}(r_m,P).\Delta_P^{mn}(r_n,P).(4\pi)^{3/2}.\varepsilon'_r.} . e^{-i2\pi f \frac{\sqrt{\varepsilon'_r}}{c}(\Delta_P^{mn}(r_m,P)+\Delta_P^{mn}(r_n,P))} \quad \text{(1bis)}$$

En effet, l'équation (1) fournit un modèle de fonction de transfert adapté à un plan situé à une profondeur $z_p$ dans un milieu homogène de permittivité de partie réelle $\varepsilon'_r$ et de partie imaginaire négligeable. Sa phase est calculée en considérant le retard accumulé par l'onde pour une propagation rectiligne entre l'antenne Tx et le diffuseur, puis entre le diffuseur et l'antenne Rx. Son amplitude est fournie par l'équation radar (voir référence [7]).

L'observation de résultats expérimentaux tend à montrer que le modèle donné par l'équation (1) n'est pas toujours optimal concernant la modélisation de l'amplitude.

Une explication physique à l'adaptation proposée par l'équation (1bis) est que la surface équivalente radar (SER) est supposée indépendante des propriétés radioélectriques du milieu. Or on estime que la SER d'un objet donné évolue de manière inversement proportionnelle à la permittivité $\varepsilon'_r$ du milieu.

Le modèle de l'équation (1bis) permet d'améliorer la focalisation, tel que cela est explicité par la suite.

La figure 3 représente un schéma de principe de cette modélisation dans un plan transversal (Ox,z). Un groupe de 8 antennes (M=N=8) est disposé dans un plan qui correspond par exemple à la surface d'un sol. Sur la figure 3 on a représenté un premier plan 301 correspondant à une séparation entre deux couches du sol de permittivités différentes et un second plan 302 qui correspond à un plan test situé à une profondeur z arbitraire selon le modèle décrit précédemment.

A l'étape 103, on détermine un coefficient de corrélation entre la mesure réalisée à l'étape 101 et le modèle déterminé à l'étape 102, pour chaque couple d'antennes.

Par exemple, le coefficient de corrélation est déterminé au moyen de la relation (3) qui représente une égalisation de type Zero Forcing (ZF).

$$\mathcal{L}_{mn}(f, z_p, \varepsilon'_r) = \frac{H_{mn}(f).W_{mn}^*(f,z_p,\varepsilon'_r)}{|W_{mn}(f,z_p,\varepsilon'_r)|^2} \quad \text{(3)}$$

* représente l'opérateur de conjugaison complexe

Alternativement, le coefficient de corrélation peut être obtenu via une autre formule d'égalisation, comme par exemple celles de type Minimum Mean Square Error (MMSE) ou Maximum Ratio Combining (MRC).

Le coefficient de corrélation dépend au moins de la fréquence, d'au moins une coordonnée d'espace et d'au moins une propriété radioélectrique et est représentatif d'une corrélation entre la fonction de transfert mesurée $H_{mn}(f)$ et le modèle $W_{mn}([f, z_p, \varepsilon'_r)$.

Les étapes 101,102,103 sont itérées pour chaque couple associant une antenne en émission d'indice m et une antenne en réception d'indice n.

A l'étape 104, on détermine ensuite une fonction d'estimation globale du coefficient de corrélation pour l'ensemble des observations réalisées par le système de détection radiofréquence.

En considérant que les fonctions de transfert sont observées sur un ensemble de L fréquences discrètes $f_l$, ainsi que pour les différents angles bi-statiques provenant de M positions $r_m$ de l'émetteur et N positions $r_n$ du récepteur, la fonction d'estimation globale est obtenue en effectuant une somme cohérente des différentes observations tel que représenté par l'équation (4) :

$$\mathcal{L}(z_p, \varepsilon'_r) = \frac{1}{MNL} \sum_{m=1}^{M} \sum_{n=1}^{N} \sum_{l=1}^{L} \mathcal{L}_{mn}(f_l, z_p, \varepsilon'_r) \quad \text{(4)}$$

A l'étape 105, on recherche ensuite au moins un maximum de la fonction d'estimation globale ou plus précisément de sa valeur absolue lorsque cette fonction est complexe. La recherche est effectuée dans l'espace multidimensionnel constitué des variables d'espace et des variables caractérisant les propriétés radioélectriques formant le domaine de définition de cette fonction.

Cette approche repose sur le principe que les valeurs de propriétés radioélectriques qui correspondent le mieux à la réalité physique du terrain fournissent des valeurs de coefficient de corrélation qui sont cohérentes entre différentes fréquences et différentes configurations d'observations, maximisant le module de la somme cohérente de l'équation (4).

Cette maximisation du module de la somme cohérente des coefficients de corrélation pour des valeurs de variables qui correspondent à une réalité physique observée est encore appelé « principe de focalisation ».

A l'étape 106 de la méthode, on déduit au moins une valeur de propriété radioélectrique associée à une position spatiale d'un plan P.

Par exemple, pour l'exemple des équations (3) et (4), on obtient une valeur de position $z_p^{max}$ et une valeur de permittivité diélectrique $\varepsilon'^{max}_r$ associée à cette position qui sont définis par l'équation (5) :

$$\left(z_p^{max}, \varepsilon'^{max}_r\right) = \underset{z_p, \varepsilon'_r}{\mathrm{argmax}}\left|\mathcal{L}\left(z_p, \varepsilon'_r\right)\right| \quad (5)$$

Argmax représente la fonction mathématique argument du maximum, qui fournit les valeurs des variables pour lesquelles une fonction atteint son maximum.

Etant donné le modèle a priori donné par l'équation (1), le vecteur $z_p^{max}$ renseigne sur la position du plan fournissant la Surface Equivalente Radar la plus importante dans l'espace observé et $\varepsilon'^{max}_r$ renseigne sur la valeur la plus représentative de la partie réelle de la permittivité diélectrique moyenne dans la partie du milieu parcourue par l'onde électromagnétique entre les antennes d'émission et les antennes de réception via le plan situé à la profondeur $z_p^{max}$.

Avantageusement, l'étape 106 n'est pas limitée à la recherche d'un seul maximum mais peut être étendue à la recherche de plusieurs maxima locaux de la fonction $|\mathcal{L}(z_p, \varepsilon'_r)|$ correspondant à plusieurs plans séparant plusieurs couches du milieu stratifié.

Par exemple, on recherche les R sommets de la fonction continue $|\mathcal{L}(z_p, \varepsilon'_r)|$ présentant les valeurs les plus élevées. Les coordonnées de chaque maximum local fournissent à la fois la position spatiale d'un diffuseur important associé à un plan et la valeur de la propriété radioélectrique la plus représentative du milieu traversé par l'onde électromagnétique entre les antennes d'émission et les antennes de réception via ce plan.

Dans une variante de réalisation de l'invention, la recherche du maximum (étape 105) peut être réalisée dans un sous-domaine du domaine de définition de la fonction $|\mathcal{L}(z_p, \varepsilon'_r)|$. Ainsi, par exemple, on peut limiter la recherche du maximum à un sous ensemble de positions spatiales données, par exemple les points situés au-delà d'une certaine profondeur dans le cas d'un radar à pénétration de sol. De même, la recherche des valeurs de propriétés radioélectriques peut être limitée à un intervalle prédéfini.

Dans une variante de réalisation de l'invention, on utilise l'algorithme de Dix décrit dans la référence [8] pour estimer les permittivités moyennes de plusieurs couches du sol.

En effet, les valeurs moyennes des permittivités estimées par la méthode de la figure 1 correspondent à la portion du sol comprise entre le réseau d'antenne et les plans diffuseurs associés aux maxima détectés.

Pour obtenir la permittivité par couche à partir de la permittivité moyenne, on applique l'algorithme de Dix qui est basé sur les vélocités en moyennes quadratiques. Cette méthode permet de décomposer les permittivités moyennes en permittivités par couches.

Ainsi, la permittivité $\varepsilon_{r[cnn]}$ de la couche $C_n$ est déterminée à partir des permittivités moyennes $\varepsilon_{r[c0→n]}$ entre le réseau d'antennes et la couche $C_n$ et entre le réseau d'antennes et la couche $C_{n-1}$ $\varepsilon_{r[c0→n-1]}$ au moyen des relation suivantes :

$$\varepsilon_{r[c_n]} = \left(\frac{z_n\sqrt{\varepsilon_{r[c_0→n]}} - z_{n-1}\sqrt{\varepsilon_{r[c_0→n-1]}}}{z_n - z_{n-1}}\right)^2 \quad (6)$$

$$\varepsilon_{r[c_0→0]} = 1$$

$$z_0 = 0$$

Dans une autre variante de réalisation, la fonction d'estimation globale $\mathcal{L}(x_p, z_p, \varepsilon'_r)$ est modifiée de sorte à introduire un changement de variable. En effet, pour faciliter la recherche de maxima dans un espace multidimensionnel, il est pertinent de modifier la variable de profondeur z en la remplaçant par une variable de profondeur électrique $z_{el} = z.\sqrt{\varepsilon_r'}$.

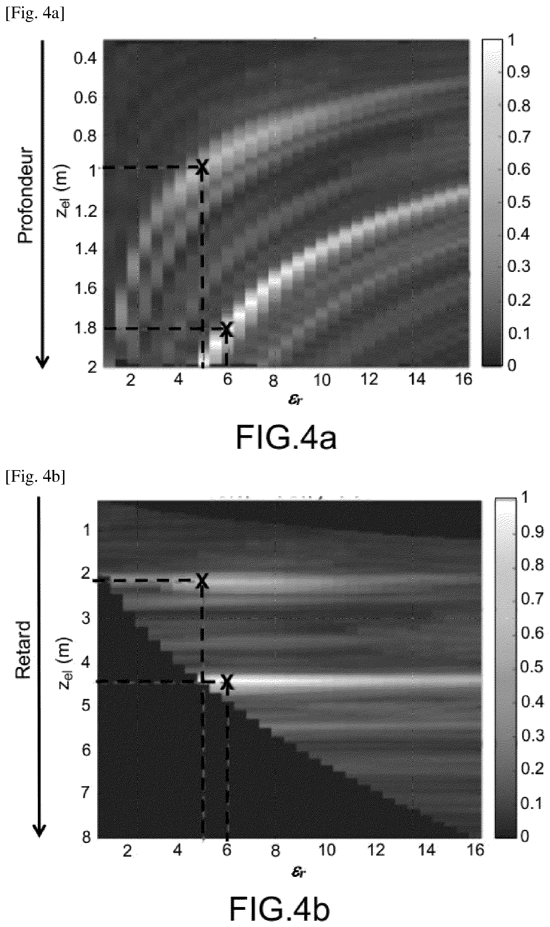

Les figures 4a et 4b illustrent, sur un exemple, l'intérêt d'un tel changement de variable.

La figure 4a représente la valeur de la fonction $\mathcal{L}(x_p, z_p, \varepsilon'_r)$ représentée par sa valeur maximale selon la dimension x dans le plan $(\varepsilon'_r, z)$.

La figure 4b représente la même valeur dans le plan $(\varepsilon'_r, z_{el})$.

On peut remarquer que la tâche de focalisation est nettement mieux définie dans le plan $(\varepsilon'_r, z_{el})$. Ceci est dû au fait que la métrique physique qui est analysée est fondamentalement de nature temporelle car homogène à un retard et non de nature spatiale. On obtient ainsi une fonction mieux quantifiée lorsqu'on l'observe sur une grille régulière du plan $(\varepsilon'_r, z_{el})$, par rapport au plan $(\varepsilon'_r, z)$.

Sur les figures 4a et 4b deux maxima sont observés pour les valeurs $(\varepsilon'_r, z_{el})$ = (5, 2.10m) et (6,4.50 m).

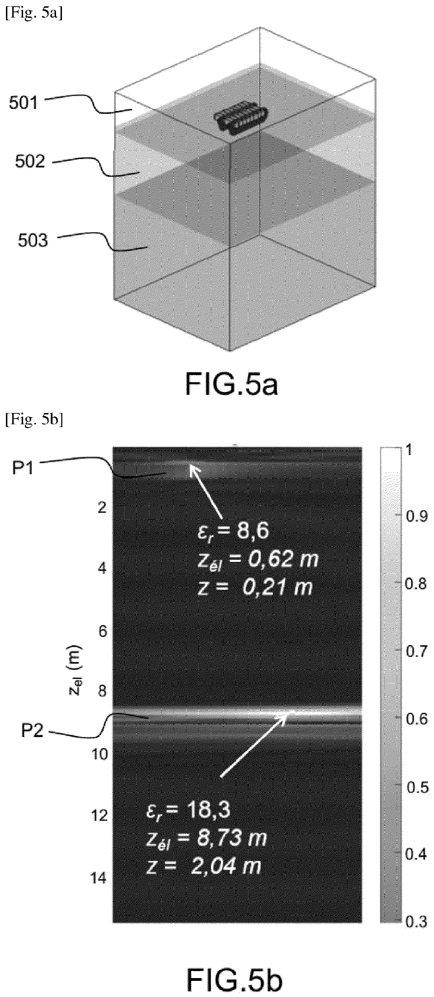

Les figures 5a et 5b illustrent un exemple de résultat de la méthode selon l'invention appliquée à la détection de plans et caractérisation de couches du sol.

La figure 5a schématise un exemple de sol stratifié composé de trois couches : une première couche d'asphalte 501 de permittivité $\varepsilon_{ra}$ = 9 entre les profondeurs $z_0$=0 m et $z_1$ = 0,15 m, une deuxième couche 502 de matériau de remblaiement de permittivité $\varepsilon_{rmr}$=20 entre les profondeurs $z_1$=0.15 m et $z_2$=1.95 m et une troisième couche 503 de sol générique de permittivité $\varepsilon_{rsg}$=15, à partir de la profondeur $z_2$=1.95 m.

Les résultats de la méthode selon l'invention sont illustrés sur la figure 5b. Un premier plan P1 est détecté à une profondeur de 21 cm proche de la profondeur réelle de 15cm de la première interface entre les deux premières couches 501 et 502. Un second plan P2 est détecté à une profondeur de 2,04m proche de la profondeur réelle de 1.95 m de la seconde interface entre les deux dernières couches 502 et 503. Les permittivités moyennes détectées par l'algorithme pour les milieux situés au-dessus de ces deux plans sont égales respectivement à 8.6 et 18.3. Après application de l'algorithme de Dix, les permittivités obtenues pour les deux premières couches sont égales respectivement à 8.6 et 20.0.

Dans une variante de réalisation de l'invention, le modèle de fonction de transfert des équations (1), (1bis) et (2) fourni pour un plan parallèle à la surface du sol et au plan du radar peut être modifié pour être adapté à la détection de plans inclinés ou obliques par rapport à la surface du sol.

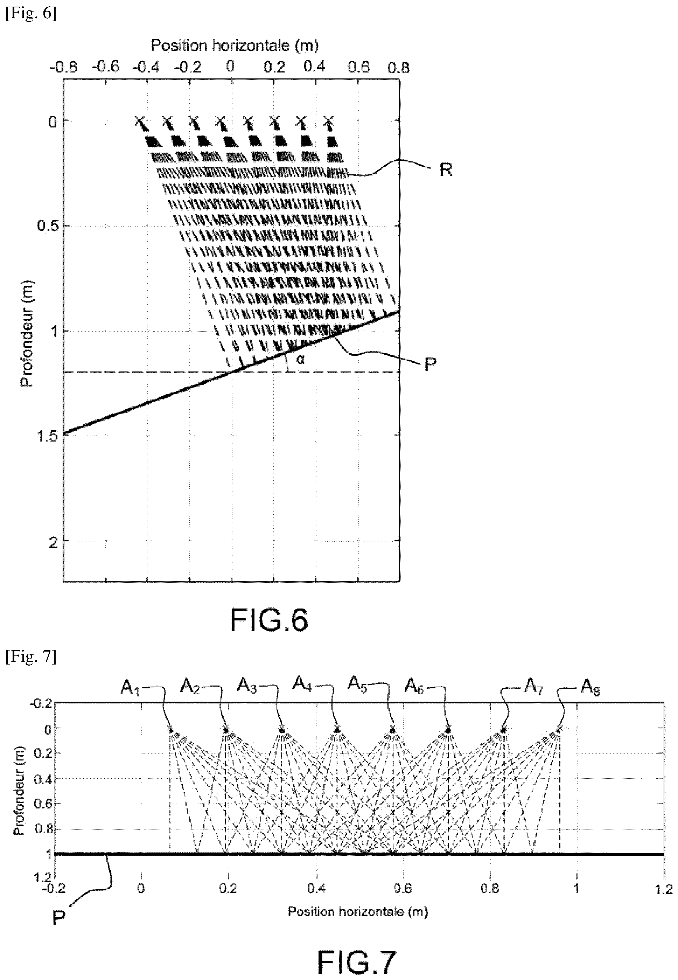

Pour cela, on définit un angle $\alpha$ que forme le plan P avec un plan horizontal parallèle à la surface du sol. Ce principe est schématisé à la figure 6. Dans cette configuration, la côte $z_p$ ne représente plus la profondeur constante du plan, mais la profondeur du plan à la verticale d'un point fixe du réseau d'antennes R, par exemple le centre du réseau d'antennes, d'abscisse xc. Par exemple, on peut fixer xc=0 sans perte de généralité.

Les distances $\Delta_P^{mn}(r_m, P)$ et $\Delta_P^{mn}(r_n, P)$ introduites dans le modèle de l'équation (1bis) se calculent alors à l'aide des relations suivantes :

$$\Delta_P^{mn}(r_m, P) = \sqrt{(x_m - x_0^{mn})^2 + z_0^{mn2}} \qquad (7)$$

$$\Delta_P^{mn}(r_n, P) = \sqrt{(x_n - x_0^{mn})^2 + x_0^{mn2}} \qquad (8)$$

Les variables suivantes sont introduites :

$$x_0^{mn} = \frac{x_n z'_m - (x_n - x'_m)z_P}{z'_m - (x_n - x'_m)\tan\alpha} \qquad (9)$$

$$z_0^{mn} = -x_0^{mn}\tan\alpha + z_p \qquad (10)$$

$$x'_m = 2z_p \cos\alpha\sin\alpha + x_m(1 - 2\sin^2\alpha) \qquad (11)$$

$$z'_m = 2\cos\alpha(z_p\cos\alpha - x_m\sin\alpha) \qquad (12)$$

$(x'_m, z'_m)$ représente les coordonnées du point image de l'antenne d'émission d'indice m par symétrie axiale via le plan P et $(x_0^{mn}, z_0^{mn})$ représente les coordonnées du point de réflexion spéculaire entre l'antenne d'émission d'indice m et l'antenne de réception d'indice n via le plan P.

Si α=0 on retombe sur les distances calculées via l'équation (2).

La figure 6 illustre les trajets de réflexion spéculaire sur un plan oblique P d'inclinaison α=30°.

En utilisant les équations (7) et (8) pour calculer les distances $\Delta_P^{mn}(r_m, P)$ et $\Delta_P^{mn}(r_n, P)$ dans l'évaluation de la fonction de transfert $W_{mn}(f)$ donnée par l'équation (1) ou (1bis), on obtient une fonction d'estimation globale $\mathcal{L}_{mn}(z_p, \alpha, \varepsilon'_r)$ à trois variables : la cote du plan oblique à la verticale d'un point fixe du réseau d'antennes, l'angle que forme le plan oblique avec le plan horizontal parallèle à la surface du sol et du plan formé par le détecteur, et la permittivité diélectrique du milieu.

La méthode de recherche des maxima de cette fonction décrite précédemment s'applique ensuite à l'identique pour déterminer les coordonnées du plan oblique ($z_p$, α) et les propriétés radioélectriques du milieu $\varepsilon'_r$.

La méthode selon l'invention est également adaptée et s'applique directement dans le cas où le milieu est constitué de strates horizontales, mais où le plan formé par le détecteur est incliné ou oblique par rapport à la surface du milieu. Un cas d'application de ce scénario est lorsque le détecteur radiofréquences est positionné dans un plan incliné par rapport à la surface du milieu. Par exemple, dans le domaine des radars à pénétration de sol, le radar peut être incliné à l'avant d'un véhicule de manière à illuminer une portion de sol de façon oblique.

Dans une autre variante de réalisation de l'invention, la fonction d'estimation globale donnée par l'équation (4) est modifiée en affectant un poids variable à chaque coefficient de corrélation. L'équation (4) devient alors :

$$\mathcal{L}(r_p, \varepsilon'_r) = \frac{1}{MNL} \sum_{m=1}^{M} \sum_{n=1}^{N} \sum_{l=1}^{L} \gamma(m, n, l) . \mathcal{L}_{mn}(f_l, r_p, \varepsilon'_r) \quad (13)$$

$\gamma(m, n, l)$ est un coefficient de pondération qui peut prendre des valeurs différentes pour chaque observation associée à une fréquence et à un couple d'antennes.

Par exemple, la fonction de pondération suivante permet de ne pas tenir compte des observations réalisées par l'antenne d'émission d'indice m=1, dans le cas où ces observations sont jugées erronées :

$$\gamma(m, n, l) = 0, si \ m = 1$$

$$\gamma(m, n, l) = 1, si \ m \neq 1$$

La figure 7 illustre un exemple de détermination des coefficients de pondération pour un cas d'application à la détection de plans horizontaux, c'est-à-dire parallèles au plan formé par les antennes du radar, par exemple le plan parallèle à la surface du sol.

La figure 7 schématise une telle configuration pour un exemple de 8 antennes $A_1$-$A_8$. On peut remarquer que plusieurs paires d'antennes (émission/réception) effectuent une observation similaire du plan P au sens où le trajet de l'onde émise par l'antenne d'émission vers le plan P, puis réfléchie vers l'antenne de réception, est le même pour plusieurs couples d'antennes.

Les couples d'antennes suivants ont ainsi la même observation du plan P et peuvent être rassemblés en 8 groupes G1-G8 :

- G1 : ($A_1, A_1$) ; ($A_2, A_2$) ; ($A_3, A_3$) ; ($A_4, A_4$) ; ($A_5, A_5$) ; ($A_6, A_6$) ; ($A_7, A_7$) ; ($A_8, A_8$)

- G2 : ($A_1, A_2$) ; ($A_2, A_3$) ; ($A_3, A_4$) ; ($A_4, A_5$) ; ($A_5, A_6$) ; ($A_6, A_7$) ; ($A_7, A_8$) ; ($A_2, A_1$) ; ($A_3, A_2$) ; ($A_4, A_3$) ; ($A_5, A_4$) ; ($A_6, A_5$) ; ($A_7, A_6$) ; ($A_8, A_7$) ;

- G3 : ($A_1, A_3$) ; ($A_2, A_4$) ; ($A_3, A_5$) ; ($A_4, A_6$) ; ($A_5, A_7$) ; ($A_6, A_8$) ; ($A_3, A_1$) ; ($A_4, A_2$) ; ($A_5, A_3$) ; ($A_6, A_4$) ; ($A_7, A_5$) ; ($A_8, A_6$) ;

- G4 : ($A_1, A_4$) ; ($A_2, A_5$) ; ($A_3, A_6$) ; ($A_4, A_7$) ; ($A_5, A_8$) ; ($A_4, A_1$) ; ($A_5, A_2$) ; ($A_6, A_3$) ; ($A_7, A_4$) ; ($A_8, A_5$)

- G5 : ($A_1, A_5$) ; ($A_2, A_6$) ; ($A_3, A_7$) ; ($A_4, A_8$) ; ($A_5, A_1$) ; ($A_6, A_2$) ; ($A_7, A_3$) ; ($A_8, A_4$) ;

- G6 : ($A_1, A_6$) ; ($A_2, A_7$) ; ($A_3, A_8$) ; ($A_6, A_1$) ; ($A_7, A_2$) ; ($A_8, A_3$) ;

- G7 : ($A_1, A_7$) ; ($A_2, A_8$) ; ($A_7, A_1$) ; ($A_8, A_2$) ;

- G8 : $(A_1, A_8)$ ; $(A_8, A_1)$ ;

Un exemple de choix de pondération est d'équilibrer l'apport de chaque type d'observation en dénombrant les paires correspondant à chaque type d'observation et en allouant à chaque paire un poids inversement proportionnel au nombre de paires similaires.

Plus généralement, le coefficient de pondération $\gamma$ de chaque groupe de couples d'antennes ayant la même observation du plan P est égal à $\gamma = k/N_p$ où $N_p$ est le nombre de paires d'antennes ayant la même observation du plan P et k est un nombre strictement positif. On donne ci-dessous les valeurs de $N_p$ pour chaque groupe :

- G1 : $N_p = 8$

- G2 : $N_p = 14$

- G3 : $N_p = 12$

- G4 : $N_p = 10$

- G5 : $N_p = 8$

- G6 : $N_p = 6$

- G7 : $N_p = 4$

- G8 : $N_p = 2$

Pour normaliser la valeur absolue de la fonction de corrélation par rapport au cas non pondéré, la valeur de k est par exemple choisie telle que $k = MxN/N_g$, où $N_g$ représente le nombre de groupes de couples d'antennes, soit un coefficient de pondération égal à $\gamma = (MxN)/(N_p x\ N_g)$. Dans l'exemple de la figure 7, $N_g = 8$.

En reprenant l'exemple de la figure 7, on alloue les poids suivants aux huit groupes définis ci-dessus :

-

$$G1 : \gamma = 64/(8{*}8) = 1$$

-

$$G2 : \gamma = 64/(14{*}8) = 4/7$$

-

$$G3 : \gamma = 64/(12{*}8) = 2/3$$

-

$$G4 : \gamma = 64/(10{*}8) = 4/5$$

-

$$G5 : \gamma = 64/(8{*}8) = 1$$

-

$$G6 : \gamma = 64/(6{*}8) = 4/3$$

-

$$G7 : \gamma = 64/(4*8)= 2$$

-

$$G8 : \gamma = 64/(2*8)= 4$$

Autrement dit, chaque coefficient de pondération est inversement proportionnel au nombre de couples d'antennes qui présentent la même disposition géométrique, par exemple la même distance cumulée, entre l'antenne d'émission, le point du plan et l'antenne de réception que celle du couple associé au coefficient de corrélation. Un autre exemple de disposition géométrique permettant de comparer les couples d'antennes est l'angle d'incidence formé entre la normale au plan et le trajet de l'antenne d'émission au point du plan.

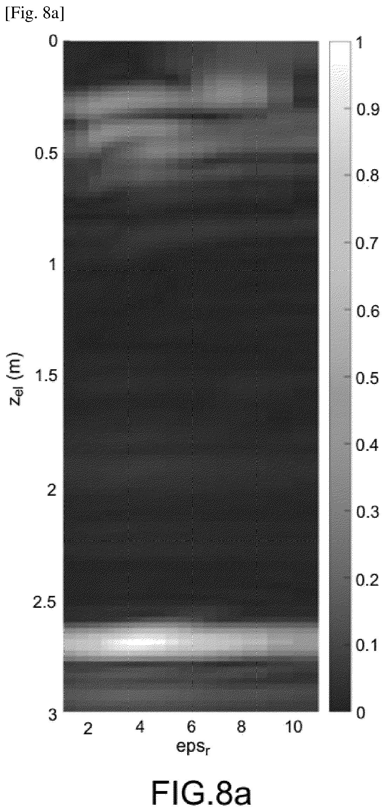

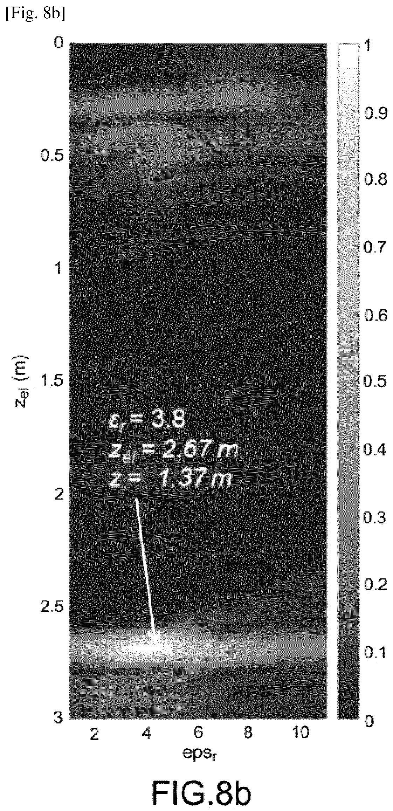

Les figures 8a et 8b présentent un résultat de focalisation appliquée à une mesure consistant à détecter un plan horizontal à une profondeur de 1.40m et à évaluer la permittivité du matériau constituant la couche correspondante formée de sable.

La figure 8a présente le résultat sans coefficients de pondération dans le calcul de la fonction d'estimation globale (équation 4). La figure 8b présente le même résultat en appliquant les coefficients de pondération décrits ci-dessus à la fonction d'estimation globale (équation 13).

On remarque que l'application des coefficients de pondération selon les positions des antennes vis-à-vis du plan permet une focalisation plus fine, permettant de mieux définir la valeur de permittivité optimisant la fonction d'estimation globale.

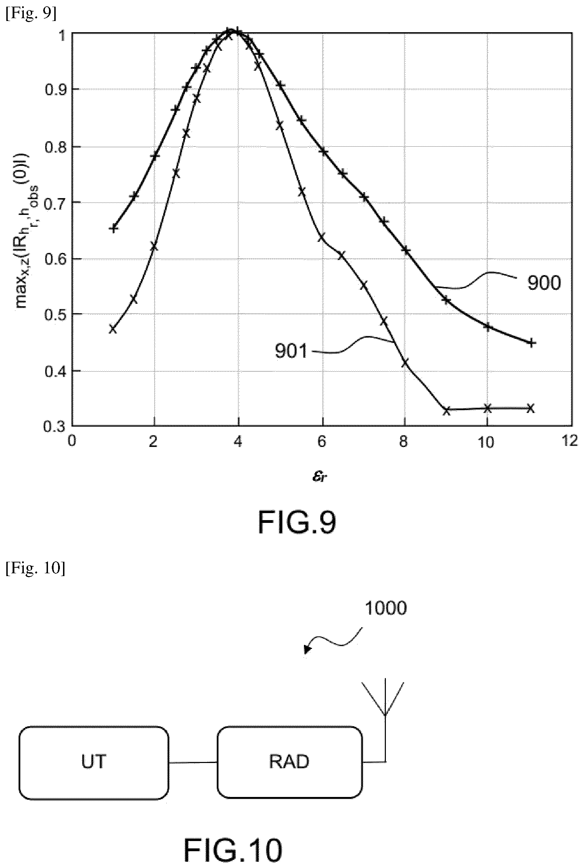

Cela peut également être vu sur la figure 9 qui représente l'allure du maximum selon la variable de profondeur z de la fonction d'estimation globale, dans les deux cas précités, en fonction de la partie réelle de la permittivité. La courbe 900 présente les résultats correspondant à la figure 8a (sans coefficients de pondération). La courbe 901 présente les résultats correspondant à la figure 8b (avec coefficients de pondération). On peut voir que la courbe 901 est davantage recentrée autour de son maximum que la courbe 900. La valeur de permittivité obtenue est donc plus précise dans le second cas.

La figure 10 représente schématiquement un système 1000 de détermination de propriétés radioélectriques d'un milieu selon un mode de réalisation de l'invention.

Le système 1000 comporte principalement un dispositif de détection radiofréquences RAD du type de celui décrit en figure 2 et une unité de traitement UT configurée pour mettre en œuvre la méthode selon l'invention à partir de mesures réalisées par le dispositif RAD.

L'unité de traitement UT peut être réalisée sous forme logicielle et/ou matérielle notamment en utilisant un ou plusieurs processeur(s) et une ou plusieurs mémoire(s). Le processeur peut être un processeur générique, un processeur spécifique, un circuit intégré propre à une application (connu aussi sous le nom anglais d'ASIC pour « Application-Specific Integrated Circuit ») ou un réseau de portes programmables in situ (connu aussi sous le nom anglais de FPGA pour « Field-Programmable Gate Array »).

Le système 1000 peut comporter une interface utilisateur pour afficher des résultats produits par la méthode.

Références

[1] S. Serkan and V. Borecky, "Estimation methods for obtaining GPR signal velocity," in Proceedings of the Third International Conference on ACSEE, Zurich, Switzerland, 2015, pp. 10-11.

[2] E. Forte and M. Pipan, "Review of multi-offset GPR applications : Data acquisition, processing and analysis," Signal processing, vol. 132, pp. 210-220, 2017.

[3] I. Dal Bo, A. Klotzsche et al., "Geophysical imaging of regolith in landscapes along a climate and vegetation gradient in the Chilean coastal cordillera," Catena, vol. 180, pp. 146-159, 2019.

[4] Q. Dou, L. Wei, D. R. Magee, and A. G. Cohn, "Real-time hyperbola recognition and fitting in GPR data," IEEE Transactions on Geoscience and Remote Sensing, vol. 55, no. 1, pp. 51-62, 2017.

[5] C. Ozdemir, S,. Demirci et al., "A review on migration methods in B-scan ground penetrating radar imaging," Mathematical Problems in Engineering, vol. 2014, 2014.

[6] Z. Dong, X. Feng et al., "3D migration depth focus velocity analysis of hand-held ground penetrating radar," Geosciences, vol. 12, no. 4, p. 178, 2022.

[7] E. F. Knott, J. F. Schaeffer, and M. T. Tulley, Radar Cross Section. SciTech Publishing, 2004.

[8] C. H. Dix, "Seismic velocities from surface measurements," Geophysics, vol. 20, no. 1, pp. 68-86,1955.[9] Demery, D. A., J. D. Parsons, and A. M. D. Turkmani. "Sounding techniques for wideband mobile radio channels: a review." IEEE Proceedings (Communications, Speech and Vision) 138.5 (1991): 437-446.

[9] Demery, D. A., J. D. Parsons, and A. M. D. Turkmani. "Sounding techniques for wideband mobile radio channels: a review." IEEE Proceedings (Communications, Speech and Vision) 138.5 (1991): 437-446.

[10] Laurenson, Dave, and Peter Grant. "A review of radio channel sounding techniques." 2006 14th European Signal Processing Conference. IEEE, 2006.

[11] Ullah, M. Habib, and A. Unggul Priantoro. "A review on multiplexing schemes for MIMO channel sounding." International Journal of Computer Science and Network Security 9.6 (2009): 294-300.

**Revendications**

1. Méthode, mise en œuvre par ordinateur, de détermination de propriétés radioélectriques d'un milieu stratifié comprenant les étapes de :

   - Déterminer (101), au moyen d'un système de détection radiofréquence comprenant au moins un émetteur et un récepteur, pour chaque couple associant un récepteur et un émetteur, au moins une mesure d'une fonction de transfert fréquentielle d'un canal de transmission caractérisant le milieu,
   - Déterminer (102) un modèle de ladite fonction de transfert fréquentielle pour un modèle du milieu composé d'au moins deux couches séparées par un plan, le modèle de la fonction de transfert étant fonction d'au moins une variable caractérisant une propriété radioélectrique du milieu et d'au moins une variable caractérisant la position du plan par rapport au système de détection,
   - Déterminer (103,104) une fonction d'estimation d'un coefficient de corrélation entre l'au moins une mesure d'une fonction de transfert fréquentielle et le modèle de ladite fonction,
   - Rechercher (105) au moins un maximum local de la fonction d'estimation sur le domaine défini par l'au moins une variable caractérisant la position du plan et l'au moins une variable caractérisant une propriété radioélectrique du milieu,
   - En déduire (106) au moins une valeur de position d'un plan et une valeur associée à ce plan de la variable caractérisant la propriété radioélectrique du milieu.

2. Méthode de détermination de propriétés radioélectriques de couches d'un milieu stratifié selon la revendication 1 dans laquelle le plan est parallèle à la surface du milieu, le système de détection est disposé parallèlement à la surface du milieu et l'au moins une variable caractérisant la position du plan comprend la profondeur du plan.

3. Méthode de détermination de propriétés radioélectriques de couches d'un milieu stratifié selon la revendication 1 dans laquelle le plan n'est pas parallèle à la surface du milieu, le système de détection est disposé parallèlement à la surface du milieu ou incliné par rapport à la surface du milieu et l'au moins une variable caractérisant la position du plan comprend la profondeur du plan par rapport à un point du système de détection et l'angle d'inclinaison du plan par rapport à la surface du milieu ou par rapport au système de détection.

4. Méthode de détermination de propriétés radioélectriques de couches d'un milieu stratifié selon l'une quelconque des revendications précédentes dans laquelle les étapes de déterminer (101) au moins une mesure de la fonction de transfert fréquentielle et de déterminer (102) un modèle de ladite fonction de transfert sont réalisées pour chaque couple associant une antenne d'émission et une antenne de réception du système de détection, la méthode comprenant en outre la détermination (103) d'un coefficient de corrélation entre ladite mesure et ledit modèle pour chacun desdits couples, la fonction d'estimation étant calculée (104) à partir de tous les coefficients de corrélation.

5. Méthode de détermination de propriétés radioélectriques de couches d'un milieu stratifié selon la revendication 4 dans laquelle l'amplitude et la phase du modèle de la fonction de transfert fréquentielle dépendent de la distance entre une antenne d'émission et un point du plan et de la distance entre le point du plan et une antenne de réception.

6. Méthode de détermination de propriétés radioélectriques de couches d'un milieu stratifié selon l'une quelconque des revendications 4 à 5 dans laquelle la fonction d'estimation du coefficient de corrélation est déterminée (104) comme une moyenne des coefficients de corrélation pour tous les couples et pour une ou plusieurs valeurs de fréquence discrètes.

7. Méthode de détermination de propriétés radioélectriques d'un milieu selon la revendication 6 dans laquelle chaque coefficient de corrélation est pondéré par un coefficient de pondération prédéfini.

8. Méthode de détermination de propriétés radioélectriques de couches d'un milieu stratifié selon la revendication 7 dans laquelle chaque coefficient de pondération est inversement proportionnel au nombre de couples qui présentent la même disposition géométrique entre l'antenne d'émission, le point du plan et l'antenne de réception que celle du couple associé au coefficient de corrélation.

9. Méthode de détermination de propriétés radioélectriques de couches d'un milieu stratifié selon l'une quelconque des revendications précédentes dans laquelle l'au moins une variable caractérisant une propriété radioélectrique du milieu est prise parmi : la partie réelle ou la partie imaginaire de la permittivité diélectrique ou de la perméabilité.

10. Méthode de détermination de propriétés radioélectriques de couches d'un milieu stratifié selon l'une quelconque des revendications précédentes dans laquelle le coefficient de corrélation est déterminé par une méthode d'égalisation de type ZF, MMSE ou MRC.

11. Méthode de détermination de propriétés radioélectriques de couches d'un milieu stratifié selon l'une quelconque des revendications 2 à 9 comprenant une étape de remplacement, dans la fonction d'estimation, de la variable de profondeur par une variable de profondeur électrique $z_{el} = z.\sqrt{\varepsilon_r'}$ , où $\varepsilon'_r$ est la partie réelle de la permittivité diélectrique.

12. Méthode de détermination de propriétés radioélectriques de couches d'un milieu stratifié selon l'une quelconque des revendications précédentes comprenant en outre l'application d'un algorithme de type Dix pour déterminer les permittivités respectives des couches du milieu à partir des permittivités estimées.

13. Méthode de détermination de propriétés radioélectriques de couches d'un milieu stratifié selon l'une quelconque des revendications précédentes dans lequel le milieu stratifié est le sol et le système de détection est un radar à pénétration de sol.

14. Dispositif (1000) de détermination de propriétés radioélectriques de couches d'un milieu stratifié comprenant un système de détection radiofréquences (RAD) comprenant au moins une antenne d'émission et une antenne de réception et une unité de traitement (UT), le dispositif étant configuré pour mettre en œuvre la méthode selon l'une quelconque des revendications précédentes.

15. Programme d'ordinateur comprenant des instructions de code qui conduisent le dispositif selon la revendication 14 à exécuter les étapes de la méthode selon l'une quelconque des revendications 1 à 13.

16. Support lisible par ordinateur, sur lequel est enregistré le programme d'ordinateur selon la revendication 15.

[Fig. 1]

FIG.1

[Fig. 2]

FIG.2

[Fig. 3]

FIG.3

[Fig. 4a]

FIG.4a

[Fig. 4b]

FIG.4b

[Fig. 5a]

FIG.5a

[Fig. 5b]

FIG.5b

[Fig. 6]

FIG.6

[Fig. 7]

FIG.7

[Fig. 8a]

FIG.8a

[Fig. 8b]

FIG.8b

[Fig. 9]

FIG.9

[Fig. 10]

FIG.10

| | Europäisches Patentamt<br>European Patent Office<br>Office européen des brevets | **RAPPORT DE RECHERCHE EUROPEENNE** | Numéro de la demande<br>EP 25 20 3776 |
|---|---|---|---|

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | US 2024/176012 A1 (MAKHOUL GLORIA [FR] ET AL) 30 mai 2024 (2024-05-30)<br>* figures 1,2 *<br>* alinéas [0002] - [0046] *<br>* alinéas [0065], [0073] *<br>* alinéas [0107] - [0129] *<br>----- | 1-16 | INV.<br>G01V3/12<br>G01S13/88<br>G01V3/15<br>G01V3/38 |
| A | US 2023/266461 A1 (D'ERRICO RAFFAELE [FR] ET AL) 24 août 2023 (2023-08-24)<br>* le document en entier *<br>----- | 1-16 | |
| A,D | WO 2022/091456 A1 (MITSUBISHI ELECTRIC CORP [JP] ET AL.) 5 mai 2022 (2022-05-05)<br>* le document en entier *<br>----- | 1-16 | |
| A | LAMBOT SÉBASTIEN ET AL: "Analysis of air-launched ground-penetrating radar techniques to measure the soil surface water content",<br>WATER RESOURCES RESEARCH.<br>,<br>vol. 42, no. 11<br>1 novembre 2006 (2006-11-01), XP093261856, US<br>ISSN: 0043-1397, DOI: 10.1029/2006WR005097<br>Extrait de l'Internet:<br>URL:https://api.wiley.com/onlinelibrary/tdm/v1/articles/10.1029%2F2006WR005097<br>* le document en entier *<br>----- | 1-16 | **DOMAINES TECHNIQUES RECHERCHES (IPC)**<br>G01V<br>G01S |

Le présent rapport a été établi pour toutes les revendications

| Lieu de la recherche | Date d'achèvement de la recherche | Examinateur |
|---|---|---|
| La Haye | 19 janvier 2026 | Parmentier, Hélène |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

**EP 4 722 764 A1**

## ANNEXE AU RAPPORT DE RECHERCHE EUROPEENNE
## RELATIF A LA DEMANDE DE BREVET EUROPEEN NO.

EP 25 20 3776

La présente annexe indique les membres de la famille de brevets relatifs aux documents  brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

19-01-2026

| Document brevet cité au rapport de recherche | Date de publication | Membre(s) de la famille de brevet(s) | | Date de publication |
|---|---|---|---|---|
| US 2024176012 A1 | 30-05-2024 | EP | 4375663 A1 | 29-05-2024 |
| | | FR | 3142553 A1 | 31-05-2024 |
| | | US | 2024176012 A1 | 30-05-2024 |
| US 2023266461 A1 | 24-08-2023 | EP | 4172655 A1 | 03-05-2023 |
| | | FR | 3111994 A1 | 31-12-2021 |
| | | US | 2023266461 A1 | 24-08-2023 |
| | | WO | 2022002700 A1 | 06-01-2022 |
| WO 2022091456 A1 | 05-05-2022 | AUCUN | | |

EPO FORM P0460

Pour tout renseignement concernant cette annexe : voir Journal Officiel de l'Office européen des brevets, No.12/82

24

## RÉFÉRENCES CITÉES DANS LA DESCRIPTION

**Documents brevets cités dans la description**

- WO 2020180191 A
- EP 3164672 A
- WO 2022091456 A

- FR 3041108
- FR 3142553


**Littérature non-brevet citée dans la description**

- **S. SERKAN** ; **V. BORECKY**. Estimation methods for obtaining GPR signal velocity. *in Proceedings of the Third International Conference on ACSEE, Zurich, Switzerland*, 2015, 10-11
- **E. FORTE** ; **M. PIPAN**. Review of multi-offset GPR applications : Data acquisition, processing and analysis. *Signal processing*, 2017, vol. 132, 210-220
- **I. DAL BO** ; **A. KLOTZSCHE et al.** Geophysical imaging of regolith in landscapes along a climate and vegetation gradient in the Chilean coastal cordillera. *Catena*, 2019, vol. 180, 146-159
- **Q. DOU** ; **L. WEI** ; **D. R. MAGEE** ; **A. G. COHN**. Real-time hyperbola recognition and fitting in GPR data. *IEEE Transactions on Geoscience and Remote Sensing*, 2017, vol. 55 (1), 51-62
- **C. OZDEMIR** ; **S,. DEMIRCI et al.** A review on migration methods in B-scan ground penetrating radar imaging. *Mathematical Problems in Engineering*, 2014, vol. 2014
- **Z. DONG** ; **X. FENG et al.** 3D migration depth focus velocity analysis of hand-held ground penetrating radar. *Geosciences*, 2022, vol. 12 (4), 178

- **E. F. KNOTT** ; **J. F. SCHAEFFER** ; **M. T. TULLEY**. Radar Cross Section. SciTech Publishing, 2004
- **C. H. DIX**. Seismic velocities from surface measurements. *Geophysics*, 1955, vol. 20 (1), 68-86
- **DEMERY, D. A** ; **J. D. PARSONS** ; **A. M. D. TURKMANI**. Sounding techniques for wideband mobile radio channels: a review. *IEEE Proceedings (Communications, Speech and Vision*, 1991, vol. 138.5, 437-446
- **DEMERY, D. A.** ; **J. D. PARSONS** ; **A. M. D. TURKMANI**. Sounding techniques for wideband mobile radio channels: a review. *IEEE Proceedings (Communications, Speech and Vision)*, 1991, vol. 138.5, 437-446
- A review of radio channel sounding techniques. **LAURENSON, DAVE** ; **PETER GRANT**. 2006 14th European Signal Processing Conference.. IEEE, 2006
- **ULLAH, M. HABIB** ; **A. UNGGUL PRIANTORO**. A review on multiplexing schemes for MIMO channel sounding. *International Journal of Computer Science and Network Security*, 2009, vol. 9.6, 294-300